# EUROPÄISCHE PATENTANMELDUNG

(11) **EP 4 552 701 A2**
(43) Veröffentlichungstag der Anmeldung: **14.05.2025**
(21) Anmeldenummer: 25167110.3
(22) Anmeldetag: 21.07.2015
(51) Int. Cl.: A61P 39/06

(54) **WASSERLÖSLICHES GRANULAT ZUR HERSTELLUNG EINER TRINKLÖSUNG**

(30) Priorität: 12.08.2014 DE 202014006415 U
(62) Teilanmeldung aus: 15744151.0
(71) Anmelder: STADA ARZNEIMITTEL AG, 61118 Bad Vilbel (DE)
(72) Erfinder: SAUERLAND, Sandra, 55216 Ingelheim am Rhein (DE); HEIGENHAUSER, Sonja, 55216 Ingelheim am Rhein (DE); SCHEURING, UWE, 55216 Ingelheim am Rhein (DE); TIMM, Oliver, 55216 Ingelheim am Rhein (DE)
(74) Vertreter: Kernebeck, Thomas

(57) **Zusammenfassung**

Die Erfindung betrifft ein wasserlösliches Granulat zur Herstellung einer Trinklösung, insbesondere zur Vorbeugung oder Behandlung von mit leichter bis mäßiger chronischer venöser Insuffizienz der unteren Extremitäten verbundenen Beschwerden enthaltend mindestens 20 Gewichtsprozente eines konzentrierten Extraktes von roten Weinblättern sowie eine gebrauchsfertige Verpackungseinheit umfassend ein oder mehrere Portionstütchen, welche das erfindungsgemäße Granulat enthalten.

## Beschreibung

### PRIORITÄTSANMELDUNG

Die vollständige Offenbarung der zu Grunde liegenden Gebrauchsmusteranmeldung DE 20 2014 006 415.6 vom 12.08.2014, deren Priorität hierin beansprucht wird, wird hiermit durch Bezugnahme einbezogen.

### HINTERGRUND DER ERFINDUNG

### 1. TECHNISCHES GEBIET

Die Erfindung betrifft ein wasserlösliches Granulat zur Herstellung einer Trinklösung, insbesondere zur Vorbeugung oder Behandlung von mit leichter bis mäßiger chronischer venöser Insuffizienz der unteren Extremitäten verbundenen Beschwerden enthaltend mindestens 20 Gewichtsprozente eines konzentrierten Extraktes von roten Weinblättern.

### 2. STAND DER TECHNIK

In dem deutschen Patent DE 600 27 481 wird eine Zusammensetzung, die in einer für die orale Verabreichung geeigneten Form ist, zur Behandlung von chronischer venöser Insuffizienz mit einem Extrakt aus Blättern von roten Weinreben vorgeschlagen.

Das deutsche Patent DE 603 32 767 beschreibt eine Filmtablette mit einem Extrakt aus roten Weinblättern.

Im EMA "Assessment Report on Vitis vinifera L., folium" (EMA/HMPC/16633/2009) vom 15. Juli 2010 werden die präklinischen und klinischen Daten bezüglich des wohl etablierten und traditionellen Einsatzes von Weinlaubblättern zusammengefasst.

Das Nahrungsergänzungsmittel "Programme Jambes Legeres" der Firma Yves Rocher ist ein Trinkgranulat, welches in neben 10 Gewichtsprozenten eines Extraktes von Hibiskus-Blüten und natürlichem Blaubeerenaroma etwa 5 Gewichtsprozente eines Extraktes von roten Weinlaubblättern enthält.

Der Gehalt an Wirksubstanz in diesem Produkt ist jedoch zu gering, um einen signifikanten Effekt bei der Vorbeugung oder Behandlung von mit leichter bis mäßiger chronischer venöser Insuffizienz der unteren Extremitäten verbundenen Beschwerden zu erzielen.

Der vorliegenden Erfindung lag die Aufgabe zu Grunde ein wohlschmeckendes, wasserlösliches Granulat enthaltend ein Extraktes von roten Weinblättern zur Herstellung einer Trinklösung, die zur Vorbeugung oder Behandlung von mit leichter bis mäßiger chronischer venöser Insuffizienz der unteren Extremitäten verbundenen Beschwerden geeignet ist, aufzuzeigen.

Eine weitere Anforderung ist eine lange Lagerstabilität des Granulats ohne wesentliche Reduktion des Gehaltes der Flavonoide des Extraktes.

Außerdem sollte es von den Verwendern hinsichtlich des Geschmackes gegebenenfalls auch ohne Zusatz eines künstlichen oder natürlichen Aromastoffes akzeptiert werden, um eine hohe Compliance zu erzielen.

Überraschenderweise wurde nun gefunden, dass ein wasserlösliches Granulat bestehend aus mindestens 20 Gewichtsprozenten eines konzentrierten Extraktes von roten Weinblättern, einem wasserlöslichen Trägermaterial, einem Säuerungsmittel, einem Fließregulierungsmittel und einem Süßungsmittel zur Herstellung einer Trinklösung, zur Vorbeugung oder Behandlung von mit leichter bis mäßiger chronischer venöser Insuffizienz der unteren Extremitäten verbundenen Beschwerden hervorragend geeignet ist.

Das Hauptmerkmal des Granulats liegt hierbei in der schnellen und kompletten Auflösung in heißen oder kalten Flüssigkeiten wie Wasser, Tee oder Fruchtsäften. Dies wird durch ein grobes Granulat poröser Struktur erreicht.

Weiterhin wurde überraschenderweise gefunden, dass das erfindungsgemäße Granulat eine hohe Antioxidationswirkung aufweist.

### KURZE ZUSAMMENFASSUNG DER ERFINDUNG

Gegenstand der Erfindung ist somit ein wasserlösliches Granulat zur Herstellung einer Trinklösung bestehend aus einem konzentrierten Extrakt von roten Weinblättern, einem wasserlöslichen Trägermaterial, einem Säuerungsmittel, einem Fließregulierungsmittel, einem Süßungsmittel und gegebenenfalls einem Aromastoff, wobei das Granulat mindestens 20 Gewichtsprozente des Extraktes von roten Weinblättern enthält.

Ein weiterer Aspekt der Erfindung ist eine Methode zur Vorbeugung oder Behandlung von mit leichter bis mäßiger chronischer venöser Insuffizienz der unteren Extremitäten verbundenen Beschwerden oder zur Verbesserung des mikrovaskulären Blutflusses, wobei diese Methode die Verabreichung einer wässrigen Trinklösung erhältlich durch
- Mischen eines Granulats bestehend
   ∘ aus einem konzentrierten Extrakt von roten Weinblättern, einem wasserlöslichen Trägermaterial, einem Säuerungsmittel, einem Fließregulierungsmittel, einem Süßungsmittel und gegebenenfalls einem Aromastoff,
   ∘ wobei das Granulat mindestens 20 Gewichtsprozente des Extraktes von roten Weinblättern enthält,
- mit Wasser oder einem wässrigen Getränk,
an eine Person umfasst.

Ein weiterer Gegenstand der Erfindung ist eine gebrauchsfertige Verpackungseinheit bestehend im Wesentlichen aus
(A) einem oder mehreren versiegelten Portionstütchen enthaltend 500 bis 1000, vorzugsweise 700 bis 900, insbesondere etwa 800 mg eines erfindungsgemäßen Granulates; und
(B) einer Packungsbeilage, in der das Öffnen der Portionstütchen (A) und die Menge an Wasser oder eines Getränkes, in dem das Granulat aufzulösen ist, sowie gegebenenfalls die Anwendung zur Vorbeugung oder Behandlung von mit leichter bis mäßiger chronischer venöser Insuffizienz der unteren Extremitäten verbundenen Beschwerden oder zur Verbesserung des mikrovaskulären Blutflusses erläutert wird.

### DETAILLIERTE BESCHREIBUNG DER ERFINDUNG

Der Begriff "konzentrierter Extrakt von roten Weinblättern", wie er vor- und nachstehend verwendet wird, bezieht sich auf einen wässrigen Extrakt von roten Blättern der Weinrebe wie er zum Beispiel in dem vorstehend erwähnten EMA Assessment Report EMA/HMPC/16633/2009 beschrieben ist.

Dieser Extrakt ist zum Beispiel erhältlich durch
(a) Sammeln von roten Weinblättern zu einem Zeitpunkt, an dem der Flavonoidgehalt ein Optimum erreicht;
(b) Trocknen und Zerkleinern der Blätter;
(c) Schneiden der Blätter in Stücke;
(d) Extrahieren der Blätter mit Wasser bei Temperaturen von 60 bis 80°C für 6 bis 10 Stunden durch vollständige Perkolation;
(e) Konzentrieren des erhaltenen Extrakts.

Insbesondere handelt es sich dabei um den dort beschriebenen Trockenextrakt von *Vitis vinifera* L., folium (4-6:1; Wasser).

Der Begriff "Granulat", wie er vor- und nachstehend verwendet wird, bezieht sich auf eine körnige bis pulverförmige, leicht schüttbare, feste Zusammensetzung, vorzugsweise eine grobe Zusammensetzung mit poröser Struktur. In der Regel wird es durch Granulieren erhalten, insbesondere durch Feuchtgranulierung, wobei die einzelnen Bestandteile aufgeschlämmt werden und dieses feuchte Material dann zerkleinert und getrocknet wird, oder durch Trockengranulierung bzw. Walzenkompaktierung, bei der ein Pulver der vorgemischten Bestandteile durch einen Spalt zwischen zwei Walzen getrieben wird. Die entstandenen Schülpen werden dann an einem Sieb wieder zerkleinert. Des Weiteren findet die Wirbelschichtgranulation, bei der einem Pulver der vorgemischten Bestandteile, welches sich auf einem Luftbett befindet, Wasser zugesetzt wird, Anwendung.

Schließlich kann man auch das erfindungsgemäße Granulat auch herstellen, indem man ein feuchtes Gemisch aller oder einen Teil der Bestandteile, insbesondere einem Gemisch des Extraktes und des wasserlöslichen Trägermaterials einem Sprühtrocknungsverfahren unterzieht.

Wird nur ein Teil der Bestandteile granuliert, können die anderen Komponenten später trocken dem ursprünglichen Granulat zugesetzt werden.

Bevorzugte Ausführungsformen des erfindungsgemäßen Granulats sind solche, wobei:
(i) es 20 bis 80, vorzugsweise 40 bis 75, insbesondere 45 bis 60 Gewichtsprozente eines getrockneten, wässrigen Extraktes von roten Weinblättern, insbesondere den Trockenextrakt von *Vitis vinifera* L., folium (4-6:1; Wasser) gemäß der EMA/HMPC/16633/2009 enthält; besonders bevorzugt enthält das Granulat bezogen auf eine Einzelportion, zum Beispiel eines Pulvertütchen (Sachet), 300 bis 500 mg, vorzugsweise 320 bis 400 mg, insbesondere 355 bis 380 mg, meist bevorzugt etwa 360 mg des Trockenextrakts von *Vitis vinifera* L., folium (4-6:1; Wasser) gemäß der EMA/HMPC/16633/2009;
(ii) das wasserlösliche Trägermaterial ein Kohlenhydratgemisch, insbesondere Maltodextrin ist, vorzugsweise liegt das Gewichtsverhältnis zwischen dem getrockneten, wässrigen Extraktes von roten Weinblättern und dem wasserlöslichen Trägermaterial bei 0,5 zu 1,0 bis 2,0 zu 1,0, insbesondere bei 0,8 zu 1,0 bis 1,2 zu 1,0;
(iii) das Säuerungsmittel eine organische Säure ausgewählt aus der Gruppe bestehend aus Äpfelsäure, Zitronensäure, Ascorbinsäure, Milchsäure und Weinsäure, insbesondere Weinsäure ist. Vorzugsweise liegt der pH-Wert der resultierenden Trinklösung bei Auflösung in neutralem Wasser unterhalb von pH 6, insbesondere bei pH 3,0 bis pH 5,5
(iv) das Fließregulierungsmittel kolloides Siliziumdioxid ist;
(v) das Süßungsmittel ein Süßstoff ausgewählt aus der Gruppe bestehend aus Acesulfam, Arpartam, Cyclamat, Neohesperidin, Saccharin, Sucralose, Steviosid und Thaumatin, insbesondere Sucralose ist;

Neben den genannten Bestandteilen kann das Granulat auch einen oder mehrere künstliche oder natürliche Aromastoffe enthalten. Bevorzugt sind Kräuter-, Tee- und Fruchtaromen, insbesondere in fester Form. Bevorzugt sind folgende Aromen: Pfefferminze, Tee, Weintraube, Brombeere, Himbeere, Heidelbeere, Walderdbeere, Kirsche, Schwarze Johannisbeere, Pflaume, Preiselbeere Grenadine und Limette, insbesondere Limette. Ganz besonders bevorzugt ist das erfindungsgemäße Granulat frei von zusätzlichen Aromastoffen.

Vorzugsweise besteht das erfindungsgemäße Granulat im Wesentlichen aus 20 bis 80 Gew.-% des getrockneten, wässrigen Extraktes von roten Weinblättern, 20 bis 80 Gew.-% eines wasserlöslichen Trägermaterials, 8 bis 20 Gew.-% eines Säuerungsmittels, 0,5 bis 1,5 Gew.-% eines Fließregulierungsmittels, 0,2 bis 0,8 Gew.-% eines Süßungsmittels und gegebenenfalss bis zu 2,0 Gew.-% eines Aromastoffes, wobei diese Bestandteile zusammen 100 Gewichtsprozente ergeben.
(vi) es im Wesentlichen besteht aus folgenden Bestandteilen:

| Gehalt in Gew.-% | Bestandteil |
|---|---|
| 20-80 | konzentrierten Extrakt von roten Weinblättern |
| 20-80 | Maltodextrin |
| 8 - 20 | Weinsäure |
| 0,5 - 1,5 | Wasserfreies kolloidales Siliziumdioxid |
| 0,2 - 0,8 | Sucralose |
| 0 - 2,0 | Limettenaroma |

wobei diese Bestandteile zusammen 100 Gewichtsprozente ergeben;
(vii) es zur Herstellung einer Trinklösung zur Vorbeugung oder Behandlung von mit leichter bis mäßiger chronischer venöser Insuffizienz der unteren Extremitäten verbundenen Beschwerden oder zur Verbesserung des mikrovaskulären Blutflusses verwendet wird;
(viii) es zur Herstellung eines Nahrungsergänzungsmittels in Form einer Trinklösung zur Verbesserung des Stoffwechsels durch Verringerung von freien Radikalen.

Vorzugsweise wird das erfindungsgemäße Trinkgranulat in portionsgerechten Pulvertütchen (Sachets) abgefüllt. In der Regel enthalten diese Sachets 500 bis 1000, vorzugsweise 700 bis 900, insbesondere etwa 800 mg des erfindungsgemäßen Granulates. Sie werden aus unbeschichtetem oder beschichtetem Aluminiumlaminat, insbesondere mit Surlyn^{®} der Fa. DuPont beschichtetem Aluminiumlaminat gefertigt und weisen eine Länge von 5,0 bis 10,0 cm, vorzugsweise 6,0 bis 8,0, insbesondere etwa 7,5 cm sowie eine Breite von 1,0 bis 3,0 cm, vorzugsweise 1,5 bis 2,5, insbesondere etwa 2,0 cm auf. Weiterhin verfügen sie an einem Ende über eine Einkerbung, die das Öffnen der Sachets erleichtert.

### Beispiel 1

Es wird ein Granulat hergestellt und in Pulvertütchen abgefüllt, wobei jedes Pulvertütchen ein Granulat bestehend aus folgenden Bestandteilen enthält:

| Bestandteil | Gehalt in mg |
|---|---|
| konzentrierter Extrakt von roten Weinblättern (4-6:1; Wasser) | 360 |
| Maltodextrin | 360 |
| Weinsäure | 75 |
| Wasserfreies kolloidales Siliziumdioxid | 8 |
| Sucralose | 5 |

Dazu wird die entsprechende Menge des Maltodextrins in kleinen Portionen zu dem Dick-Extrakt von roten Weinlaubblättern gegeben und miteinander gemischt. Die feuchte Mischung wird in einem Sprühtrockner zu einem trockenen Granulat versprüht.

Anschließend wird das Granulat mit den übrigen Bestandteilen gemischt und abgefüllt.

Das in einem Pulvertütchen enthaltene Trinkgranulat löst sich innerhalb von 30 bis 40 sec. in 100-200 ml heisser oder kalter Flüssigkeit wie Wasser, Tee oder Fruchtsaft auf. Die erhaltene Trinklösung wurde in einem in Italien durchgeführten Konsumtentest von allen Probanden als geschmacklich angenehm und wohlschmeckend empfunden und eignet sich hervorragend die mit leichter bis mäßiger chronischer venöser Insuffizienz der unteren Extremitäten verbundenen Beschwerden zu lindern.

### Beispiel 2

Es wird ein Granulat hergestellt und in Pulvertütchen abgefüllt, wobei jedes Pulvertütchen ein Granulat bestehend aus folgenden Bestandteilen enthält:

| Bestandteil | Gehalt in mg |
|---|---|
| konzentrierter Extrakt von roten Weinblättern (4-6:1; Wasser) | 360 |
| Maltodextrin | 360 |
| Weinsäure | 75 |
| Wasserfreies kolloidales Siliziumdioxid | 8 |
| Sucralose | 5 |
| Limettenaroma | 8 |

Dazu wird die entsprechende Menge des Maltodextrins in kleinen Portionen zu dem Dick-Extrakt von roten Weinlaubblättern gegeben und miteinander gemischt. Die feuchte Mischung wird in einem Sprühtrockner zu einem trockenen Granulat versprüht.

Anschließend wird das Granulat mit den übrigen Bestandteilen gemischt und abgefüllt.

Das in einem Pulvertütchen enthaltene Trinkgranulat löst sich schnell in 100-200 ml heißer oder kalter Flüssigkeit wie Wasser, Tee oder Fruchtsaft auf. Die erhaltene Trinklösung wurde in einem in Italien durchgeführten Konsumententest von allen Probanden als geschmacklich sehr angenehm und wohlschmeckend empfunden und eignet sich hervorragend die mit leichter bis mäßiger chronischer venöser Insuffizienz der unteren Extremitäten verbundenen Beschwerden zu lindern.

### Beispiel 3

### Konsumententest

202 Probanden erhielten das erfindungsgemäße Trinkgranulat (102 erhielten Granulat gemäß Beispiel 1, 100 erhielten Granulat gemäß Beispiel 2) und wurden nach der Verkostung befragt, inwieweit Ihnen der Geschmack des resultierenden Getränkes gefalle. Dabei wurden die folgenden Ergebnisse erzielt:

| Geschmack | Beispiel 1 | Beispiel 2 |
|---|---|---|
| Sehr gut | 13 | 14 |
| Gut | 70 | 71 |
| Weder gut noch schlecht | 14 | 12 |
| Schlecht | 4 | 2 |
| Sehr schlecht | 1 | 1 |

### Beispiel 4

### Untersuchung zur antioxidativen Wirkung

Der ORAC(=Oxygen Radical Absorbance Capacity)-Test ist ein standardisiertes Verfahren zur Bestimmung der Fähigkeit zum Abfangen von Sauerstoffradikalen von Naturstoffen und wird in Trolox-Äquivalenten (µmol TE/100 g) angegeben. Das Messprinzip ist eine Messung der Fluoreszenz des Farbstoffs Fluorescein bis zu dessen Erschöpfung durch einen künstlichen Radikalenerzeuger. Ein zugegebenes Antioxidans verzögert die Oxidation des Fluoresceins und je höher die Kapazität ist, desto grösser ist die gemessene Fläche unter der Zeitkurve (AUC). Eine Beschreibung findet sich in zum Beispiel in Wikipedia unter http://www.en.wikipedia.org/wiki/Oxygen_radical_absorbance_capacity Für die Durchführung der ORAC-Tests wurde das hierfür akkreditierte Institut Prof. Kurz GmbH in 50933 Köln beauftragt ( www.institut-kurz.de ) und führte die Messungen mittels fluorimetrischen Mikrotiterplattentests IK2002-hydrophil durch.

In einer Testreihe im März 2015 wurde das erfindungsgemäße Granulat mit 360 mg Wirkprinzip gegenüber anderen handelsüblichen Fertigprodukten getestet. Die Ergebnisse sind in der folgenden Tabelle wiedergeben:

| Produkt | ORAC-Wert [µmol TE/100 g] |
|---|---|
| Erfindungsgemäßes Granulat (Charge 1) | 170500 |
| Erfindungsgemäßes Granulat (Charge 2) | 177900 |
| Pycnogenol^{®} (Pinienrindenextrakt) 50 mg GPH Kapseln, Hecht-Pharma GmbH, PZN 09188092 | 161700 |
| Vitamin C 200 mg Tabletten, medphano Arzneimittel GmbH, PZN 04588935 | 108000 |

Der ORAC-Wert der beiden erfindungsgemäßen Proben liegt in der selben Größenordnung und ist sogar noch ein wenig höher als der von Pycnogenol^{®}, das als eines der am besten untersuchten Antioxidantien gilt. Vitamin C, das ebenfalls als Antioxidans verwendet wird, hat einen um ca. 40% niedrigeren ORAC-Wert.

## Patentansprüche

1. Ein wasserlösliches Granulat zur Herstellung einer Trinklösung bestehend aus einem konzentrierten Extrakt von roten Weinblättern, einem wasserlöslichen Trägermaterial, einem Säuerungsmittel, einem Fließregulierungsmittel, einem Süßungsmittel und gegebenenfalls einem Aromastoff, **dadurch gekennzeichnet, dass** das Granulat mindestens 20 Gewichtsprozente des Extraktes von roten Weinblättern enthält.

2. Ein Granulat nach Anspruch 1, **dadurch gekennzeichnet, dass** der Extrakt von roten Weinblättern erhältlich ist durch ein Verfahren, welches die folgenden Schritte umfasst:
(a) Sammeln von roten Weinblättern zu einem Zeitpunkt, an dem der Flavonoidgehalt ein Optimum erreicht;
(b) Trocknen und Zerkleinern der Blätter;
(c) Schneiden der Blätter in Stücke;
(d) Extrahieren der Blätter mit Wasser bei Temperaturen von 60 bis 80°C für 6 bis 10 Stunden durch vollständige Perkolation;
(e) Konzentrieren des erhaltenen Extrakts.

3. Ein Granulat nach Anspruch 1 oder 2, **dadurch gekennzeichnet, dass** es 20 bis 80 Gewichtsprozente des getrockneten, wässrigen Extraktes von roten Weinblättern enthält.

4. Ein Granulat nach einem der Ansprüche 1 bis 3, **dadurch gekennzeichnet, dass** das wasserlösliche Trägermaterial ein Kohlenhydratgemisch, insbesondere Maltodextrin ist.

5. Ein Granulat nach einem der Ansprüche 1 bis 4, **dadurch gekennzeichnet, dass** das Säuerungsmittel eine organische Säure ausgewählt aus der Gruppe bestehend aus Äpfelsäure, Zitronensäure, Ascorbinsäure, Milchsäure und Weinsäure, insbesondere Weinsäure ist.

6. Ein Granulat nach einem der Ansprüche 1 bis 5, **dadurch gekennzeichnet, dass** das das Fließregulierungsmittel kolloides Siliziumdioxid ist.

7. Ein Granulat nach einem der Ansprüche 1 bis 6, **dadurch gekennzeichnet, dass** das Süßungsmittel ein Süßstoff ausgewählt aus der Gruppe bestehend aus Acesulfam, Arpartam, Cyclamat, Neohesperidin, Saccharin, Sucralose, Steviosid und Thaumatin, insbesondere Sucralose ist.

8. Ein Granulat nach einem der Ansprüche 1 bis 7, **dadurch gekennzeichnet, dass** es im Wesentlichen besteht aus folgenden Bestandteilen:
| Gehalt in Gew.-% | Bestandteil |
|---|---|
| 20-80 | konzentrierten Extrakt von roten Weinblättern |
| 20-80 | Maltodextrin |
| 8 - 20 | Weinsäure |
| 0,5 - 1,5 | Wasserfreies kolloidales Siliziumdioxid |
| 0,2 - 0,8 | Sucralose |
| 0 - 2,0 | Limettenaroma |
wobei diese Bestandteile zusammen 100 Gewichtsprozente ergeben.

9. Ein Granulat nach einem der Ansprüche 1 bis 8 zur Herstellung einer Trinklösung zur Vorbeugung oder Behandlung von mit leichter bis mäßiger chronischer venöser Insuffizienz der unteren Extremitäten verbundenen Beschwerden oder zur Verbesserung des mikrovaskulären Blutflusses.

10. Ein Granulat nach einem der Ansprüche 1 bis 8 zur Herstellung eines Nahrungsergänzungsmittels in Form einer Trinklösung zur Verbesserung des Stoffwechsels durch Verringerung von freien Radikalen.

11. Eine gebrauchsfertige Verpackungseinheit bestehend im Wesentlichen aus
(A) einem oder mehreren versiegelten Portionstütchen enthaltend 500 bis 1000, vorzugsweise 700 bis 900, insbesondere etwa 800 mg eines Granulates gemäß eines oder mehrerer der Ansprüche 1 bis 9; und
(B) einer Packungsbeilage, in der das Öffnen der Portionstütchen (A) und die Menge an Wasser oder eines Getränkes, in dem das Granulat aufzulösen ist, sowie gegebenenfalls die Anwendung zur Vorbeugung oder Behandlung von mit leichter bis mäßiger chronischer venöser Insuffizienz der unteren Extremitäten verbundenen Beschwerden oder zur Verbesserung des mikrovaskulären Blutflusses erläutert wird.
